(19) Europäisches Patentamt
European Patent Office
Office européen des brevets

(11) Veröffentlichungsnummer: **0 253 256 A1**

# (12) EUROPÄISCHE PATENTANMELDUNG

(21) Anmeldenummer: **87109693.9**

(22) Anmeldetag: **06.07.87**

(51) Int. Cl.4: **C07D 417/04**, A01N 43/78

(30) Priorität: **09.07.86 JP 159595/86**

(43) Veröffentlichungstag der Anmeldung: **20.01.88 Patentblatt 88/03**

(84) Benannte Vertragsstaaten: **BE CH DE FR GB IT LI NL**

(71) Anmelder: **NIHON TOKUSHU NOYAKU SEIZO K.K.**
**Itohpia Nihonbashi Honcho Building 7-1, Nihonbashi Honcho 2-chome**
**Chuo-ku Tokyo 103(JP)**

(72) Erfinder: **Kume, Toyohiko**
**6-7-8, Asahigaoka**
**Hino-shi Tokyo(JP)**
Erfinder: **Goto, Toshio**
**3454-21, Honmachida**
**Machida shi Tokyo(JP)**
Erfinder: **Kamochi, Atsumi**
**2-24-10, Higashi-Toyoda**
**Hino-shi Tokyo(JP)**
Erfinder: **Yanagi, Akihiko**
**2-1200-1, Nagafuchi**
**Ome-shi Tokyo(JP)**
Erfinder: **Hayakawa, Hidenori**
**1631-13, Shimokuzawa**
**Sagamihara-shi Kanagawa-ken(JP)**
Erfinder: **Yagi, Shigeki**
**1-30-7, Izumi**
**Suginami-ku Tokyo(JP)**

(74) Vertreter: **Schumacher, Günter, Dr. et al**
**c/o Bayer AG Konzernverwaltung RP Patentabteilung**
**D-5090 Leverkusen 1 Bayerwerk(DE)**

(54) **Neue N-Benzothiazolyl-2,5-dihydropyrrole.**

(57) Offenbart werden neue N-Benzothiazolyl-2,5-dihydropyrrole der Formel (I)

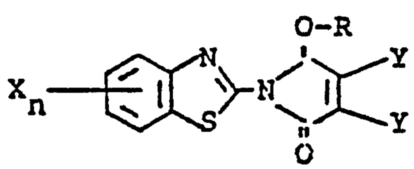

(I)

in der

R ein Wasserstoff-Atom oder eine Acyl-Gruppe,

X ein Halogen-Atom, eine Alkyl-Gruppe, eine Halogenoalkyl-Gruppe, eine Halogenoalkoxy-Gruppe oder

EP 0 253 256 A1

eine Phenyl-Gruppe
n 0, 1 oder 2 und
Y eine Alkyl-Gruppe
bezeichnen, Verfahren zu ihrer Herstellung und die Verwendung der neuen Verbindungen als Herbizide.

## Neue N-Benzothiazolyl-2,5-dihydropyrrole

Die vorliegende Erfindung betrifft neue N-Benzothiazolyl-2,5-dihydropyrrole, Verfahren zu ihrer Herstellung und ihre Verwendung als Herbizide.

Es wurde bereits offenbart, daß bestimmte 3,4-Dimethyl-2-hydroxy-5-oxo-2,5-dihydropyrrol-Derivate herbizide Aktivitäten besitzen (siehe die JP-OS 23965/1977).

Nunmehr wurden neue N-Benzothiazolyl-2,5-dihydropyrrole der Formel (I)

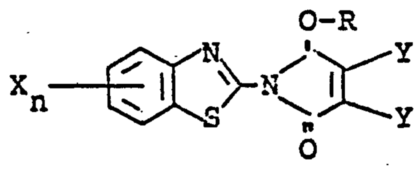

(I)

gefunden. In der Formel bezeichnen

R ein Wasserstoff-Atom oder eine Acyl-Gruppe,

X ein Halogen-Atom, eine Alkyl-Gruppe, eine Halogenoalkyl-Gruppe, eine Halogenoalkoxy-Gruppe oder eine Phenyl-Gruppe

n 0, 1 oder 2 und

Y eine Alkyl-Gruppe.

Die Verbindungen der Formel (I) werden mittels eines Verfahrens erhalten, bei dem

(a) in dem Fall, in dem R ein Wasserstoff-Atom ist, Verbindungen der Formel (II)

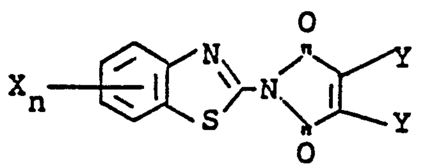

(II) ,

worin X, n und Y die angegebenen Bedeutungen haben, mit einem Reduktionsmittel in Gegenwart inerter Lösungsmittel zur Reaktion gebracht werden oder

(b) in dem Fall, in dem R eine Acyl-Gruppe ist, Verbindungen der Formel (I-a)

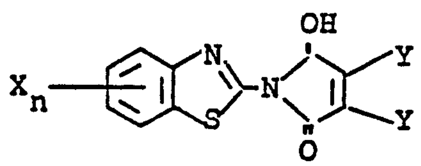

(I-a) ,

worin X, n und Y die angegebenen Bedeutungen haben, mit Verbindungen der Formel (III)

R'-Hal (III),

in der R' für eine Acyl-Gruppe steht und Hal für ein Halogen-Atom steht, oder mit Verbindungen der Formel (IV)

R'-O-R' (IV),

in der R' die angegebene Bedeutung hat, in Gegenwart inerter Lösungsmittel, und gegebenenfalls in Gegenwart säurebindender Mittel zur Reaktion gebracht werden.

Die neuen N-Benzothiazolyl-2,5-dihydropyrrole zeigen potente herbizide Eigenschaften.

Überraschenderweise zeigen die erfindungsgemäßen N-Benzothiazolyl-2,5-dihyropyrrole nicht nur eine wesentlich stärkere herbizide Wirkung als die aus dem oben genannten Stand der Technik bekannten, sondern auch eine günstige Verträglichkeit mit Nutzpflanzen, ohne Phytotoxizität.

Vorzugsweise bezeichnen in der die Verbindungen der vorliegenden Erfindung repräsentierenden Formel (I)

R ein Wasserstoff-Atom oder eine Acetyl-Gruppe,

X ein Fluor-Atom, ein Chlor-Atom, ein Brom-Atom, eine Alkyl-Gruppe mit 1 bis 3 Kohlenstoff-Atomen, eine

fluoro-substituierte Alkyl-Gruppe mit 1 bis 3 Kohlenstoff-Atomen, eine fluoro-substituierte Alkoxy-Gruppe mit 1 bis 3 Kohlenstoff-Atomen oder eine Phenyl-Gruppe,

n 0, 1 oder 2 und

Y eine Methyl-oder Ethyl-Gruppe.

Besonders bevorzugt bezeichnen in der Formel (I)

R ein Wasserstoff-Atom,

X ein Fluor-Atom, ein Chlor-Atom, eine Methyl-, Ethyl-, n-Propyl-oder Isopropyl-Gruppe, eine Trifluoromethyl-Gruppe oder eine Difluoromethoxy-, Trifluoromethoxy-oder Tetrafluoroethoxy-Gruppe,

n 0, 1 oder 2 und

Y eine Methyl-oder Ethyl-Gruppe.

Zu speziellen Beispielen für die Verbindungen der Formel (I) zählen

N-(2-Benzothiazolyl)-3,4-dimethyl-2-hydroxy-5-oxo-2,5-dihydropyrrol,

N-(6-Fluoro-2-benzothiazolyl)-3,4-dimethyl-2-hydroxy-5-oxo-2,5-dihydropyrrol,

N-(6-Trifluoromethoxy-2-benzothiazolyl)-3,4-dimethyl-2-hydroxy-5-oxo-2,5-dihydropyrrol,

N-(5-Trifluoromethyl-2-benzothiazolyl)-3,4-dimethyl-2-hydroxy-5-oxo-2,5-dihydropyrrol und

N-(6-Isopropyl-2-benzothiazolyl)-3,4-dimethyl-2-hydroxy-5-oxo-2,5-dihydropyrrol,

Wenn in dem Verfahren a) beispielsweise N-(2-Benzothiazolyl)-2,3-dimethyl-maleinimid und Natriumborhydrid als Ausgangsstoffe eingesetzt werden, wird die Reaktion durch das folgende Reaktionsschema dargestellt.

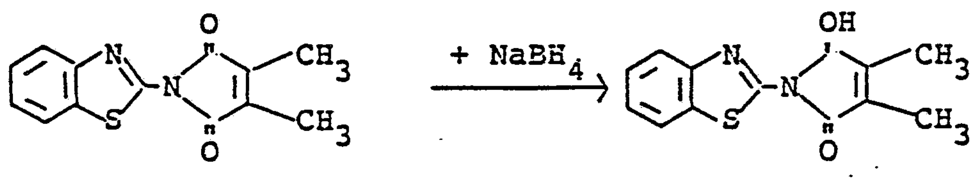


Wenn in dem Verfahren b) beispielsweise N-(6-Fluoro-2-benzothiazolyl)-3,4-dimethyl-2-hydroxy-5-oxo-2,5-dihydropyrrol und Acetylchlorid als Ausgangsstoffe eingesetzt werden, wird die Reaktion durch das folgende Scheme dargestellt.

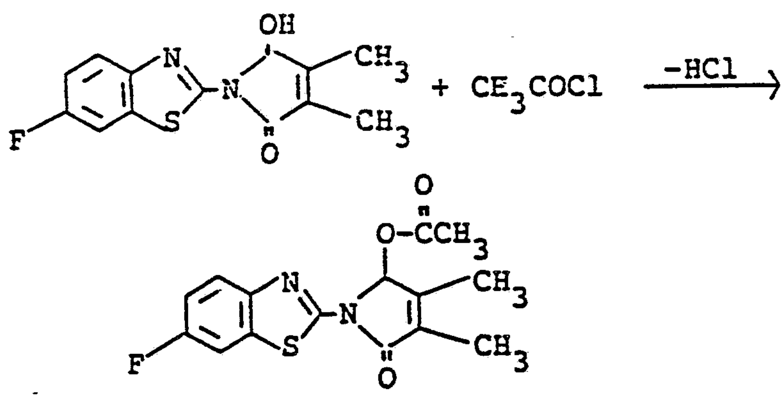


In dem Verfahren a) bezeichnet die Verbindung der Formel (II) als Ausgangsstoff eine auf den obigen Definitionen für X, n und Y basierende Verbindung, vorzugsweise eine, die auf den oben angegebenen bevorzugten Definitionen beruht.

Die Verbindungen der Formel (II) sind neue Verbindungen und können beispielsweise erhalten werden durch Umsetzung von Verbindungen der Formel (V)

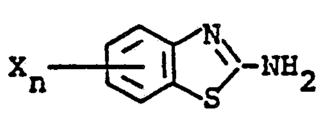

(V) ,

in der X und n die oben angegebenen Bedeutungen haben, mit Verbindungen der Formel (VI)

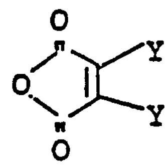

(VI) ,

in der Y die oben angegebenen Bedeutungen hat, oder mit 2 mol Maleinsäureanhydrid auf 1 mol der Verbindungen der Formel (V).

Die Verbindungen der Formel (V) unfassen neue und bekannte Verbindungen.

Die Verbindungen der Formel (V) können beispielsweise erhalten werden durch Cyclisierung von Anilinen der Formel (VII)

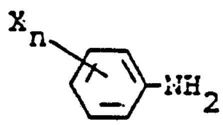

(VII) ,

worin X und n die oben angegebenen Bedeutungen haben, wobei nach der in der FR-PS 1 502 178 beschriebenen Methode Brom und Thiocyanat eingesetzt werden, oder durch oxidative Cyclisierung von Thioharnstoffen der Formel (VIII)

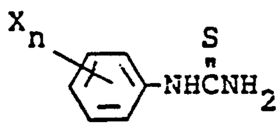

(VIII) ,

worin X und n die oben angegebenen Bedeutungen haben, nach dem in Org. Synthesis, Band 22, Seiten 16-19, und in der JP-OS 59679/1984 beschriebenen Verfahren oder durch Umsetzung von Verbindungen der Formel (IX)

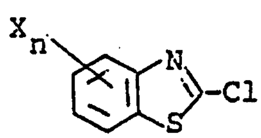

(IX) ,

mit Ammoniak nach der Methode aus "Yakugaku Zasshi", Band 78, Seiten 437-438.

Die Verbindungen der Formel (VII) sind auf dem Gebiet der organischen Chemie wohlbekannt, und die Verbindungen der Formel (VIII) lassen sich leicht durch Reaktion der Aniline der Formel (VII) mit Isothiocyansäure erhalten. Die Verbindungen der Formel (IX) sind bekannte Verbindungen, die beispielsweise in der JP-OS 70678/1984 oder in der EP-OS 0 043 573 beschrieben sind.

Die Verbindungen der Formel (VI) in Verfahren a) sind in der JP-OS 23965/1978 beschrieben, und ihr typisches Beispiel ist 2,3-Dimethylmaleinsäureanhydrid.

Andere 2,3-disubstituierte Maleinsäureandryride als 2,3-Dimethylmaleinsäureanhydrid, beispielsweise 2,3-Diethylmaleinsäureanhydrid, können durch Befolgen der Methoden erhalten werden, die in J. Japan. Chem. Soc., Band 9 (1983), Seiten 1291-1293 und Summary of Papers Read in the 50th Annual Meeting of the Japanese Chemical Society 1986, Vol. II, Seite 825, beschrieben sind. Alternativ kann 2,3-Dimethylmaleinsäureanhydrid durch Hydrolyse von Diethyl-2,3-diethyl-maleat, das in Synthetic Communications, Band 14 (1984), No. 13., Seiten 1193-1198, beschrieben ist, in üblicher Weise und Umsetzung des Produkts mit Acetylchlorid erhalten werden. Außerdem können 2,3-disubstituierte Maleinsäureandyride mittels des Verfahrens hergestellt werden, das in J. Org. Chem., Band 38 (1973), Seiten 3386-3389, beschrieben ist.

Zu speziellen Beispielen für die Verbindungen der Formel (II) zählen
N-(2-Benzothiazolyl)-dimethylmaleinimid,
N-(6-Fluoro-2-benzothiazolyl)-dimethylmaleinimid,
N-(6-Trifluoromethoxy-2-benzothiazolyl)-dimethylmaleinimid und
N-(5-Trifluoromethyl-2-benzothiazolyl)-dimethylmaleinimid.

Beispiele für das in Verfahren a) eingesetzte Reduktionsmittel sind Metallhydride wie Natriumborhydrid und Lithiumaluminiumhydrid.

Die als Ausgangsstoffe in dem Verfahren b) eingesetzten Verbindungen der Formel (I-a) zählen zu den durch die vorliegende Erfindung verfügbar gemachten Verbindungen der Formel (I), die mittels des Verfahrens a) hergestellt werden können.

Die Verbindungen der Formeln (III) oder (IV), die in gleicher Weise Ausgangsstoffe sind, bezeichnen auf den obigen Definitionen für R' und Hal basierende Verbindungen, vorzugsweise solche, worin R' eine Acetyl-Gruppe ist und Hal ein Chlor-Atom ist.

Acetylchlorid, das als spezielles Beispiel für die Verbindungen der Formel (III) angeführt wird, ist wohlbekannt. Die Verbindungen der Formel (IV) sind ebenfalls wohlbekannt, und Acetanhydrid kann als spezielles Beispiel genannt werden.

Bei der praktischen Durchführung des Verfahrens a) können sämtliche inerten organischen Lösungsmittel als geeignete Verdünnungsmittel verwendet werden.

Beispiele für die Verdünnungsmittel sind Wasser, Ether (wie Dioxan und Tetrahydrofuran), Alkohole (wie Methanol, Ethanol, Isopropanol, Butanol und Ethylenglycol) und Säureamide (wie Dimethylformamid).

Das Verfahren a) kann innerhalb eines Temperaturbereichs von beträchtlicher Breite, beispielsweise bei einer Temperatur zwischen etwa -20 °C und etwa 80 °C, vorzugsweise zwischen etwa 0 °C und etwa 30 °C, durchgeführt werden.

Zweckmäßigerweise wird die Reaktion unter Atmosphärendruck durchgeführt, jedoch ist es auch möglich, unter erhöhtem oder vermindertem Druck zu arbeiten.

Bei der Durchführung des Verfahrens a) kann die gewünschte Verbindungen der Formel (I) in einfacher Weise durch Reduktion der Verbindung der Formel (II) mit einem Reduktionsmittel, wie sie oben beispielhaft genannt sind, erhalten werden, wie in den nachstehend angegebenen Beispielen gezeigt wird.

Für die praktische Durchführung des Verfahrens b) seien Ether (wie Dioxan und Tetrahydrofuran), Säureamide (wie Dimethylformamid, Dimethylacetamid und Hexamethylphosphorsäuretriamid) und Sulfoxide (wie Dimethylsulfoxid) als Beispiele für geeignete Verdünnungsmittel angeführt.

Das Verfahren b) kann in Gegenwart eines säurebindenden Mittels durchgeführt werden. Beispiele für die säurebindenden Mittel sind Hydride, Hydroxide, Carbonate, Hydrogencarbonate und Alkoholate von Alkalimetallen und tertiäre Amine wie Triethylamin, Diethylanilin, Pyridin und 4-Dimethylaminopyridin.

Das Verfahren b) kann innerhalb eines Temperaturbereichs von beträchtlicher Breite, beispielsweise bei einer Temperatur zwischen etwa 50 °C und etwa 200 °C, vorzugsweise zwischen etwa 80 °C und etwa 160 °C, durchgeführt werden.

Zweckmäßigerweise wird die Reaktion unter Atmosphärendruck durchgeführt, jedoch ist es auch möglich, unter erhöhtem oder vermindertem Druck zu arbeiten.

Bei der praktischen Durchführung des Verfahrens b) können die gewünschten Verbindung der Formel (I) beispielsweise durch Umsetzung von 1 mol der Verbindungen der Formel (I-a) mit 1 bis etwa 1,5 mol der Verbindungen der Formel (III) oder (IV) in einem inerten Lösungsmittel, gegebenenfalls in Gegenwart eines säurebindenden Mittels, umgesetzt werden.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Die aktiven Verbindungen gemäß der Erfindung können beispielsweise in Verbindung mit den folgenden Pflanzen verwendet werden:

<u>Dikotyledonen-Unkräuter der Gattungen:</u>

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

Dikotyledonen-Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita.

Monokotyledonen-Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen:

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplatagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können in übliche Präparate wie Lösungen, Emulsionen, Suspensionen, Pulver, Stäubemittel, Schäume, Pasten, lösliche Pulver, Granulat, Aerosole, mit dem Wirkstoff imprägnierte natürliche und synthetische Materialien, sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie ULV-Kaltvernebelungs- und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Unter verflüssigten gasförmigen Verdünnungsmitteln oder Trägern sind Flüssigkeiten zu verstehen, die bei normaler Temperatur und unter normalem Druck gasförmig sein würden, beispielsweise Aerosol-Treibmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe oder Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Präparate auch zur Unkrautbekämpfung als Mischungen mit anderen Herbiziden eingesetzt werden, wobei Fertigpräparate oder Tankmischungen möglich sind.

Mischungen mit anderen aktiven Verbindungen wie Herbiziden, Fungiziden, Insektiziden, Akariziden, Nematiziden, vogelabweisenden Mitteln, Pflanzennährstoffen sowie Mitteln zur Verbesserung der Bodenstruktur sind ebenfalls möglich.

Die Wirkstoff können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können vor oder nach dem Auflaufen der Pflanzen zur Anwendung gelangen.

Sie können auch vor der Einsaat in den Boden eingearbeitet werden. Insbesondere werden sie nach dem Auflaufen der Pflanzen angewandt.

Die eingesetzten Mengen der aktiven Verbindung können innerhalb eines Bereichs von erheblicher Breite variiert werden. Sie hängen wesentlich von der Art der angestrebten Wirkung ab. Im allgemeinen liegen die aufgewandten Mengen des Wirkstoffs zwischen etwa 0,05 und etwa 5 kg/ha, vorzugsweise zwischen etwa 0,1 und etwa 2,5 kg/ha.

Die Herstellung und Verwendung der erfindungsgemäßen Verbindungen sind den folgenden Beispielen zu entnehmen.

Herstellungsbeispiele

BEISPIEL 1

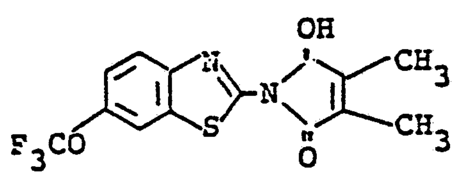

(Verbindung Nr. 4)

N-(6-Trifluoromethoxy-2-benzothiazolyl)-dimethylmaleinimid (3,42 g) wurde in Methanol (150 ml) vorgelegt, und Natriumborhydrid (0,19 g) wurde bei Raumtemperatur im Laufe von 15 min zugegeben. Die Mischung wurde 3 h nachgerührt. Nach der Reaktion wurde die Reaktionsmischung mit Essigsäure neutralisiert, und Methanol wurde unter vermindertem Druck abgedampft. Der Rückstand wurde mit Wasser aufgenommen und mit Chloroform extrahiert. Die Chloroform-Extrakte wurden vereinigt und über wasserfreiem Magnesiumsulfat getrocknet, und das Lösungsmittel wurde unter vermindertem Druck abgedampft. Der feste Rückstand wurde mit einer kleinen Menge Diethylether gewaschen, wonach das gewünschte N-(6-Trifluoromethoxy-2-benzothiazolyl)-3,4-dimethyl-2-hydroxy-5-oxo-2,5-dihydropyrrol (3,05 g) erhalten wurde; Schmp. 228-231 °C.

BEISPIEL 2

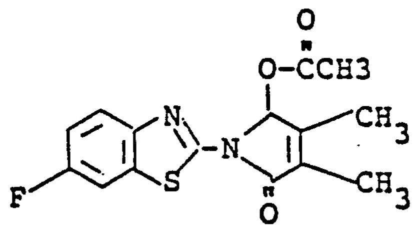

(Verbindung Nr. 13)

N-(6-Fluoro-2-benzothiazolyl)-3,4-dimethyl-2-hydroxy-5-oxo-2,5-dihydropyrrol (1,39 g) wurde zu Tetrahydrofuran (30 ml) und Natriumhydrid (0,18 g) bei Raumtemperatur hinzugefügt. Die Mischung wurde 15 min zum Rückfluß erhitzt und dann auf Raumtemperatur abgekühlt. Eine Lösung von Acetylchlorid (0,79 g) in Tetrahydrofuran (10 ml) wurde tropfenweise zugegeben. Nachdem die Mischung noch 3 h unter Rückfluß erhitzt worden war, wurde das Lösungsmittel abgedampft. Der Rückstand wurde mit Wasser vermischt und mit Chloroform extrahiert. Der Chloroform-Extrakt wurde über wasserfreiem Magnesiumsulfat getrocknet und unter vermindertem Druck zur Trockne eingedampft. Der feste Rückstand wurde mit einer kleinen Menge Diethylether gewaschen, wonach das gewünschte N-(6-Fluoro-2-benzothiazolyl)-2-oxo-3,4-dimethyl-5-acetyloxy-2,5-dihydropyrrol (1,42 g) erhalten wurde; Schmp. 180-183 °C.

Die nachstehende Tabelle 1 faßt Verbindungen der Formel (I) gemäß der vorliegenden Erfindung zusammen, die mittels ähnlichen Arbeitsweisen wie in den obigen Beispielen hergestellt werden können, gemeinsam mit den in den Beispielen 1 und 2 erhaltenen Verbindungen.

Tabelle 1

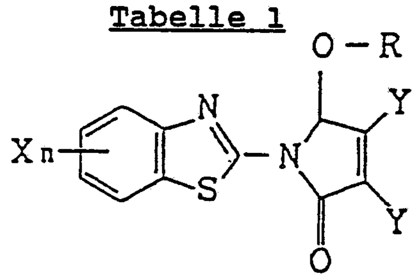

| Verb. Nr. | R | X | n | Y | Schmp.(°C) |
|---|---|---|---|---|---|
| 1 | H | − | 0 | $-CH_3$ | 183~184 |
| 2 | H | 6−F | 1 | $-CH_3$ | 215~216 |
| 3 | H | 6−Cl | 1 | $-CH_3$ | 221~223 |
| 4 | H | $6-OCF_3$ | 1 | $-CH_3$ | 228~231 |
| 5 | H | $6-OCHF_2$ | 1 | $-CH_3$ | 203~206 |
| 6 | H | $6-OCF_2CHF_2$ | 1 | $-CH_3$ | 195~197 |
| 7 | H | 6− 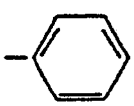 | 1 | $-CH_3$ | 178~180 |
| 8 | H | $6-C_3H_7-iso$ | 1 | $-CH_3$ | 133~135 |
| 9 | H | $6-CH_3$ | 1 | $-CH_3$ | 203~205 |
| 10 | H | $5-CF_3$ | 1 | $-CH_3$ | 220~223 |
| 11 | H | 7−Cl | 1 | $-CH_3$ | 228~231 |
| 12 | H | $5,7-Cl_2$ | 2 | $-CH_3$ | 196~198 |
| 13 | 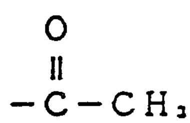 | 6−F | 1 | $-CH_3$ | 180~183 |
| 14 | H | 5−Cl | 1 | $-CH_3$ | 178~181 |
| 15 | H | 6−F | 1 | $-C_2H_5$ | 95 |
| 16 | H | 5−F | 1 | $-CH_3$ | 212~213 |

BEISPIEL 3

Synthese eines Ausgangsstoffes:

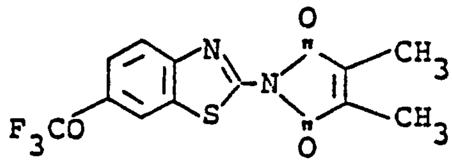

Ein 100 ml-Kolben wurde mit 2-Amino-6-trifluoromethoxybenzothiazol (7,03 g), 2,3-Dimethylmaleinsäureanhydrid (4,16 g) und Diphenylether (50 ml) beschickt, und ein Dean-Stark-Abscheider wurde auf den Kolben aufgesetzt. Die Reaktionspartner wurden etwa 5 min zum Rückfluß erhitzt. Man ließ die Reaktionsmischung abkühlen und goß sie dann in Hexan (200 ml). Die entstandenen Kristalle wurden durch Filtration gesammelt und mit einer kleinen Menge Diethylether gewaschen, wonach N-(6-Trifluoromethoxy-2-benzothiazolyl)-dimethylmaleinimid (7,66 g) erhalten wurden; Schm. 196-198 °C.

BEISPIEL 4

Synthese eines Ausgangsstoffes nach einer alternativen Methode:

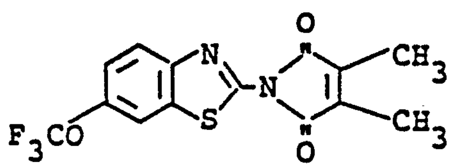

Nach der gleichen Arbeitsweise wie in Beispiel 3 wurde aus 2-Amino-6-trifluoromethoxybenzothiazol (2,34 g), Maleinsäureanhydrid (2,34 g) und Diphenylether (30 ml) die gleiche Verbindung (1,87 g) gebildet, wie sie in Beispiel 3 erhalten wurde; Schmp. 196-198 °C.

Biologische Tests

(Vergleichs-Verbindungen)

E-1:

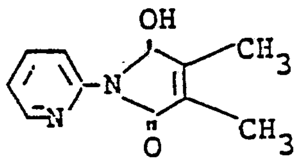

E-2:

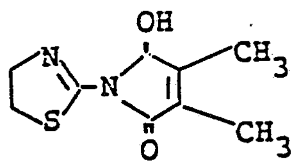

E-1 und E-2 sind Verbindungen, die in der JP-OS 23965/1978 beschrieben sind

BEISPIEL 5

Test gegen Unkräuter in einem überfluteten Reisfeld durch Wasseroberflächen-Anwendung

Herstellung eines Wirkstoff-PräparatsTräger: 5 Gewichtsteile Aceton;
Emulgator: 1 Gewichtsteil Benzyloxypolyglycolether.

Ein Wirkstoff-Präparat in Form eines emulgierbaren Konzentrats wurde erhalten durch Vermischen von 1 Gewichtsteil der aktiven Verbindung mit den oben angegebenen Mengen des Trägers und des emulgierenden Mittels. Eine vorher festgelegte Menge des Präparats wurde mit Wasser verdünnt.

Test-Verfahren

Reis-Kulturboden wurde in Töpfe (5 $dm^2$; 25 cm × 20 cm × 9 cm) gefüllt, und Reis-Setzlinge (Varietät: "Nihonbare") im 2,5-Blattstadium (15 cm hoch) wurden jeweils an zwei Stellen pro Topf als Stock von drei Setzlingen umgepflanzt. Samen von Hühnerhirse (Echinochloa oryzicola Vasing.), Monochoria (Monochoria vaginalis) sowie einjährigen breitblättrigen Unkräutern [Falsche Pimpernelle (Lindernia pyxidaria L.), Rotala indica, Amerikanischer Wasserwurz (Elatine triandra), Rotstengel (Ammannia multiflora Roxburgh) und Dopatrium junceum Hamilton] wurden eingesät, und die Töpfe wurden feucht gehalten. Zwei Tage später wurden die Töpfe bis zu einer Tiefe von etwa 2 bis 3 cm überflutet. Fünf Tage nach dem Umpflanzen der Setzlinge wurde die Verbindung der vorliegenden Erfindung, in Form eines wie im Vorstehenden hergestellten emulgierbaren Konzentrats mittels einer Pipette in einer vorher festgelegten Menge auf die Oberfläche des Wassers aufgebracht. Danach wurde der Zustand der etwa 3 cm tiefen Überflutung aufrechterhalten, und vier Wochen nach der chemischen Behandlung wurden die herbizide Wirkung und die Phytotoxizität gegenüber Reis untersucht und mit Hilfe der folgenden Skala von 0 bis 5 bewertet:

| Bewertung | Herbizide Wirkung (bewertet als Unkrautvernichtungs-Verhältnis, bezogen auf eine unbehandelte Fläche) |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |

| Bewertung | Phytotoxizität gegenüber Reis (bewertet unter Bezug auf die unbehandelte Fläche) |
|---|---|
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Test-Ergebnisse sind anhand typischer Beispiele in Tabelle 2 aufgeführt.

## Tabelle 2

| Verbindung Nr. | Wirkstoff-Menge kg/ha | Herbizide Wirkung Hühnerhirse | Monochoria | Breitblättrige Unkräuter | Phytotoxizität gegen Reis |
|---|---|---|---|---|---|
| 1 | 2 | 5 | 5 | 5 | 0 |
| | 1 | 5 | 5 | 5 | 0 |
| 2 | 2 | 5 | 5 | 5 | 0 |
| | 1 | 5 | 5 | 5 | 0 |
| 4 | 2 | 4 | 5 | 5 | 0 |
| 8 | 2 | 4 | 5 | 5 | 0 |
| 10 | 2 | 4 | 5 | 5 | 0 |
| Vergleich | | | | | |
| E-1 | 2 | 2 | 3 | 3 | 2 |
| E-2 | 2 | 3 | 3 | 4 | 5 |

BEISPIEL 6

Test gegen Unkräuter auf höher gelegenem Ackerboden durch Behandlung vor dem Auflaufen:

In einem Gewächshaus wurden Reis und Mais in Töpfe von 500 $cm^2$ Fläche, die mit Ackererde gefüllt waren, eingesät, und Erde, die Samen von grauem Amaranth (Amaranthus lividus L.) und Gänsefuß - (Chenopodium album L.) enthielt, wurde über die Erde in den Töpfen in einer Tiefe von 1 cm gestreut.

Einen Tag nach der Einsaat wurde eine Test-Chemikalie in einer vorher festgelegten Konzentration, hergestellt wie in Beispiel 5, gleichmäßig über die Oberflächenschicht des Bodens in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 5 geprüft. Die Ergebnisse sind anhand typischer Beispiele in Tabelle 3 aufgeführt.

Tabelle 3

| Verbindung Nr. | Wirkstoff-Menge kg/ha | Herbizide Wirkung Grauer Amaranth | Herbizide Wirkung Gänsefuß | Phytotoxizität gegen Reis | Phytotoxizität gegen Mais |
|---|---|---|---|---|---|
| 4 | 2 | 5 | 5 | 0 | 0 |
| | 1 | 5 | 5 | 0 | 0 |
| 10 | 2 | 5 | 5 | 1 | 0 |
| | 1 | 5 | 5 | 0 | 0 |
| Vergleich | | | | | |
| E-1 | 2 | 2 | 3 | 2 | 1 |
| | 1 | 1 | 1 | 1 | 0 |
| E-2 | 2 | 3 | 3 | 3 | 2 |
| | 1 | 1 | 2 | 2 | 1 |

BEISPIEL 7

Test gegen Unkräuter auf höher gelegenem Ackerboden durch Laub-Behandlung:

In einem Gewächschaus wurden Reis und Mais in Töpfe von 500 $cm^2$ Fläche, die mit Ackererde gefüllt waren, eingesät, und Erde, die Samen von grauem Amaranth (Amaranthus lividus L.) und Gänsefuß - (Chenopodium album L.) enthielt, wurde über die Erde in den Töpfen in einer Tiefe von 1 cm gestreut.

Nach der Einsaat wurden die Pflanzen 14 Tage angezogen, und eine Test-Chemikalie in einer vorher festgelegten Konzentration, hergestellt wie in Beispiel 5 wurde gleichmäßig über Test-Pflanzen in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 5 geprüft. Die Ergebnisse sind anhand typischer Beispiele in Tabelle 4 aufgeführt.

Tabelle 4

| Verbindung Nr. | Wirkstoff-Menge kg/ha | H e r b i z i d e W i r k u n g Grauer Amaranth | Gänsefuß | Phytotoxizität gegen Reis | Mais |
|---|---|---|---|---|---|
| 2 | 1 | 5 | 5 | 2 | 1 |
| | 0,5 | 5 | 5 | 0 | 0 |
| 4 | 1 | 5 | 5 | 0 | 0 |
| | 0,5 | 4 | 5 | 0 | 0 |
| 8 | 1 | 5 | 5 | 0 | 0 |
| | 0,5 | 5 | 5 | 0 | 0 |
| 10 | 1 | 5 | 5 | 0 | 0 |
| | 0,5 | 5 | 5 | 0 | 0 |
| Vergleich | | | | | |
| E-1 | 1 | 3 | 3 | 3 | 3 |
| | 0,5 | 1 | 2 | 2 | 1 |
| E-2 | 1 | 4 | 4 | 4 | 4 |
| | 0,5 | 3 | 3 | 3 | 2 |

## Ansprüche

1. N-Benzothiazolyl-2,5-dihydropyrrole der Formel (I)

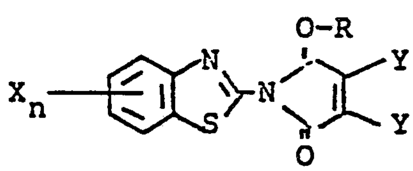

(I)

in der
R ein Wasserstoff-Atom oder eine Acyl-Gruppe,
X ein Halogen-Atom, eine Alkyl-Gruppe, eine Halogenoalkyl-Gruppe, eine Halogenoalkoxy-Gruppe oder eine Phenyl-Gruppe
n 0, 1 oder 2 und
Y eine Alkyl-Gruppe
bezeichnen.

2. Verbindungen nach Anspruch 1, worin
R ein Wasserstoff-Atom oder eine Acetyl-Gruppe,
X ein Fluor-Atom, ein Chlor-Atom, ein Brom-Atom, eine Alkyl-Gruppe mit 1 bis 3 Kohlenstoff-Atomen, eine fluoro-substituierte Alkyl-Gruppe mit 1 bis 3 Kohlenstoff-Atomen, eine fluoro-substituierte Alkoxy-Gruppe mit 1 bis 3 Kohlenstoff-Atomen oder eine Phenyl-Gruppe,
n 0, 1 oder 2 und
Y eine Methyl-oder Ethyl-Gruppe
bezeichnen.

3. Verbindungen nach Anspruch 1, worin
R ein Wasserstoff-Atom,
X ein Fluor-Atom, ein Chlor-Atom, eine Methyl-, Ethyl-, n-Propyl-oder Isopropyl-Gruppe, eine Trifluoromethyl-Gruppe oder eine Difluoromethoxy-, Trifluoromethoxy-oder Tetrafluoroethoxy-Gruppe,
n 0, 1 oder 2 und
Y eine Methyl-oder Ethyl-Gruppe
bezeichnen.

4. N-(2-Benzothiazolyl)-3,4-dimethyl-2-hydroxy-5-oxo-2,5-dihydropyrrol nach Ansprüchen 1 bis 3, dargestellt durch die nachstehende Formel

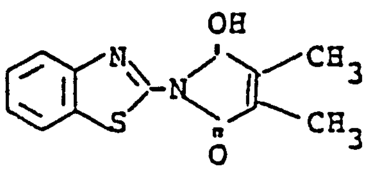

5. N-(6-Fluoro-2-benzothiazolyl)-3,4-dimethyl-2-hydroxy-5-oxo-2,5-dihydropyrrol nach Ansprüchen 1 bis 3, dargestellt durch die nachstehende Formel

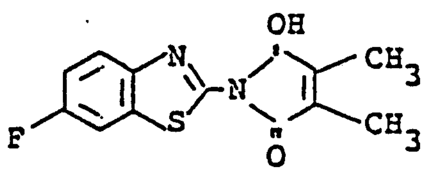

6. Verfahren zur Herstellung von N-Benzothiazolyl-2,5-dihydropyrrolen der Formel (I)

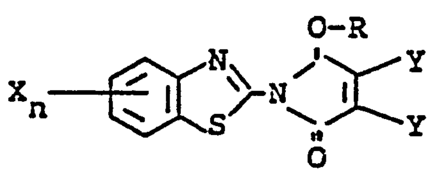

in der
R ein Wasserstoff-Atom oder eine Acyl-Gruppe,
X ein Halogen-Atom, eine Alkyl-Gruppe, eine Halogenoalkyl-Gruppe, eine Halogenoalkoxy-Gruppe oder eine Phenyl-Gruppe
n 0, 1 oder 2 und
Y eine Alkyl-Gruppe
bezeichnen,
dadurch gekennzeichnet, daß
(a) in dem Fall, in dem R ein Wasserstoff-Atom ist, Verbindungen der Formel (II)

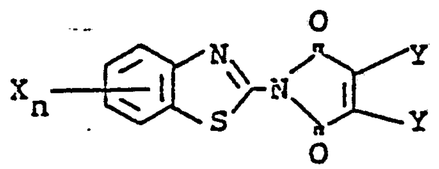

(II)

,

worin X, n und Y die angegebenen Bedeutungen haben, mit einem Reduktionsmittel in Gegenwart inerter Lösungsmittel zur Reaktion gebracht werden oder

(b) in dem Fall, in dem R eine Acyl-Gruppe ist, Verbindungen der Formel (I-a)

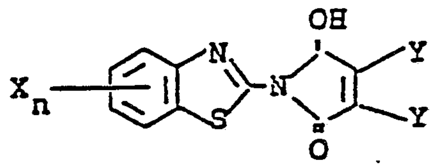

(I-a)

,

worin X, n und Y die angegebenen Bedeutungen haben, mit Verbindungen der Formel (III)

R'-Hal (III),

in der R' für eine Acyl-Gruppe steht und Hal für ein Halogen-Atom steht, oder mit Verbindungen der Formel (IV)

R'-O-R' (IV),

in der R' die angegebene Bedeutung hat, in Gegenwart inerter Lösungsmittel, und gegebenenfalls in Gegenwart säurebindender Mittel zur Reaktion gebracht werden.

7. Herbizide Mittel, dadurch gekennzeichnet, daß sie wenigstens ein N-Benzothiazolyl-2,5-dihydropyrrol der Formel (I) gemäß Anspruch 1 enthalten.

8. Verfahren zur Bekämpfung von Unkräutern, dadurch gekennzeichnet, daß N-Benzothiazolyl-2,5-dihydropyrrole der Formel (I) gemäß Anspruch 1 auf Unkräuter und/oder deren Lebensraum zur Einwirkung gebracht werden.

9. Verwendung von N-Benzothiazolyl-2,5-dihydropyrrolen der Formel (I) gemäß Anspruch 1 zur Bekämpfung von Unkräutern.

10. Verfahren zur Herstellung herbizider Zusammensetzungen, dadurch gekennzeichnet, daß N-Benzothiazolyl-2,5-dihydropyrrole der Formel (I) gemäß Anspruch 1 mit Streckmitteln und/oder grenzflächenaktiven Mitteln vermischt werden.

Europäisches Patentamt

# EUROPÄISCHER RECHERCHENBERICHT

Nummer der Anmeldung

EP 87 10 9693

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.4) |
|---|---|---|---|
| D,X | US-A-4 220 655 (BEAT BÖHNER)<br>* Ansprüche; Spalte 1, Zeilen 50-68 *<br>--- | 1-3,7-10 | C 07 D 417/04<br>A 01 N 43/78 |
| A | FR-A-2 450 257 (VELSICOL CHEMICAL CORP.)<br>* Ansprüche 1,9,10 *<br>--- | 1,7-10 | |
| A | EP-A-0 032 879 (CIBA-GEIGY)<br>* Ansprüche 1,15-19 *<br>----- | 1,7-10 | |
| | | | **RECHERCHIERTE SACHGEBIETE (Int. Cl.4)** |
| | | | C 07 D 417/00<br>C 07 D 277/00<br>C 07 D 207/00 |

Der vorliegende Recherchenbericht wurde für alle Patentansprüche erstellt

| Recherchenort | Abschlußdatum der Recherche | Prüfer |
|---|---|---|
| DEN HAAG | 06-10-1987 | HENRY J.C. |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : alteres Patentdokument, das jedoch erst am oder nach dem Anmeldedatum veröffentlicht worden ist
D : in der Anmeldung angeführtes Dokument
L : aus andern Gründen angefzhrtes Dokument

& : Mitglied der gleichen Patentfamilie, übereinstimmendes Dokument

EPO FORM 1503 03.82 (P0403)